(19) Europäisches Patentamt
European Patent Office
Office européen des brevets

(11) Veröffentlichungsnummer: **0 001 144**
**B1**

(12) # EUROPÄISCHE PATENTSCHRIFT

(45) Veröffentlichungstag der Patentschrift: **17.06.81**

(21) Anmeldenummer: **78200166.3**

(22) Anmeldetag: **29.08.78**

(51) Int. Cl.$^3$: **C 07 C 109/14, A 61 K 31/15**

(54) **N-Substituierte 2-Hydrazono-Propionsäure-Derivate, Verfahren zur Herstellung derselben und Arzneimittel, die diese enthalten.**

(30) Priorität: **01.09.77 DE 2739380**

(43) Veröffentlichungstag der Anmeldung:
**21.03.79 Patentblatt 79/6**

(45) Bekanntmachung des Hinweises auf die Patenterteilung:
**17.06.81 Patentblatt 81/24**

(84) Benannte Vertragsstaaten:
**BE CH DE FR GB LU NL SE**

(56) Entgegenhaltungen:
**DE - A - 2 643 303**

**CHEMISCHES ZENTRALBLATT, II, 362 (1911)**

**THE MERCK INDEX, Eighth Edition, 1968, MERCK & CO. Rahway New York, USA Seite 645, Abschnitt "Mebanazine"**

**THE MERCK INDEX, Eighth Edition, 1968, MERCK & CO., Rahway New York, USA Seite 806, Abschnitt "Phenelzine"**

(73) Patentinhaber: **BOEHRINGER MANNHEIM GMBH**
**Sandhofer Strasse 112-132 Postfach 31 01 20**
**D-6800 Mannheim 31-Waldhof (DE)**

(72) Erfinder: **Haeckel, Rainer, Prof. Dr.**
**Helstorferstrasse 19**
**D-3000 Hannover 61 (DE)**
Erfinder: **Oellerich, Michael, Dr.**
**Helstorferstrasse 25**
**D-3000 Hannover 61 (DE)**
Erfinder: **Heerdt, Ruth, Dr.**
**Wallstädter Strasse 62**
**D-6800 Mannheim-Feudenheim (DE)**
Erfinder: **Hübner, Manfred, Dr.**
**Saarlandstrasse 85 e**
**D-6700 Ludwigshafen (DE)**
Erfinder: **Schmidt, Felix Helmut, Prof. Dr.**
**Stockacher Strasse 1**
**D-6800 Mannheim-Seckenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 001 144 B1**

Courier Press, Leamington Spa, England.

N-Substituierte 2-Hydrazono-Propionsäure-Derivate, Verfahren zur Herstellung derselben und Arzneimittel, die diese enthalten

Est ist bekannt, daß einige Monoaminoxidasehemmer, z.B. Phenelzin (2-Phenylethyl-hydrazin) oder Mebanazin (1-Phenyl-ethyl-hydrazin) in hoher Dosierung hypoglykämisch wirksam sein können (Adnitt, P.I.: Hypoglycemic action of monoamino oxidase inhibitors, Diabetes 17: 628—633 (1968), Wickström, L., Petterson, K.: Treatment of diabetics with monoamino oxidase inhibitors, Lancet 2: 995—997 (1964), Cooper, A.J., Reddie, K.M.G.: Hypotensive collapse and hypoglycaemia after mebanazine — a monoamine-oxidase inhibitor, Lancet 1: 1133 (1964)).

Die Hauptwirkung ist jedoch die Inhibitorwirkung der Monoaminoxidase (MAO), so daß diese Verbindungen zwar in der Therapie von psychischen Erkrankungen Verwendung gefunden haben (Van Praag, H.M.: Leijnse, B.: The influence of some antidepressives of the hydrazine type on the glucose metabolism in depressed patients, Clin. Chim. Acta 8: 466—475 (1963)), aber als blutzuckersenkende Medikamente nicht eingesetzt werden konnten.

Es stellte sich deshalb die Aufgabe, Verbindungen zu finden, die eine hypoglykämische Wirkung in einem Dosisbereich zeigen, in dem die MAO-Hemmung nicht oder nur unwesentlich auftritt.

Überraschenderweise konnte gefunden werden, daß Hydrazone der Brenztraubensäure mit einer vom Phenelzin abweichenden Hydrazinkomponente gegenüber den entsprechenden Hydrazinen eine erheblich verstärkte hypoglykämische Wirkung zeigen, während die MAO-hemmende Wirkung fast vollständig zurückgedrängt wird. So besitzt z.B. die 2-(Octylhydrazono)-propionsäure im pharmakologischen Test beim Meerschweinchen eine Schwellendosis von 5 mg/kg (i.p. verabreicht), während die Schwellendosis beim bekannten Phenelzin größer als 50 mg/kg ist. Als Schwellendosis wird diejenige Dosis bezeichnet, die beim Versuchstier die Blutglucosekonzentration gegenüber einem mit einem äquivalenten Volumen physiologischer Kochsalzlösung behandelten Tier signifikant senkt.

Gegenstand der vorliegenden Erfindung sind deshalb neue Proprionsäure-Derivate der allgemeinen Formel I

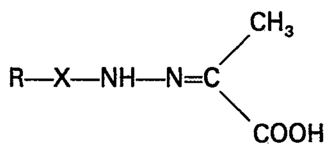

(I),

in der

R ein geradkettiger, verzweigter oder cyclischer, gesättigter oder ungesättigter Alkyl-Rest und

X ein Valenzstrich oder eine geradkettige oder verzweigte, gesättigte oder ungesättigte Alkylen-Gruppe mit 1—4 Kohlenstoffatomen ist,

wobei für den Fall, daß X einen Valenzstrich darstellt, R kein gesättigter Cycloalkyl-Rest sein darf, deren physiologisch unbedenkliche Salze, Ester und Amide, Verfahren zur Herstellung derselben sowie ihre Verwendung zur Herstellung von Arzneimitteln mit blutzuckersenkender Wirkung.

Die vom Patentanspruch ausgeklammerte Verbindung 2-(Cyclohexyl-hydrazono)-propionsäure ist literaturbekannt (Chem. Zentral. Bl. 1911, II, 362).

Pharmakologische Untersuchungen zeigten jedoch, daß diese Substanz im Vergleich zu den erfindungsgemäßen Verbindungen unwirksam ist.

Die geradkettigen oder verzweigten, gesättigten oder ungesättigten Alkylreste des Substituenten R können 1—18 Kohlenstoffatome enthalten, wobei als geradkettige, gesättigte Alkylreste der Methyl-, Ethyl-, Propyl-, Butyl-, Pentyl-, Hexyl-, Heptyl-, Octyl-, Nonyl-, Decyl-, Undecyl-, Dodecyl-, Tridecyl- und Tetradecyl-Rest in Frage kommen. Bevorzugte verzweigte, gesättigte Alkylreste sind der Isopropyl-, Isobutyl-, sec.-Butyl-, tert.-Butyl-, Isopentyl-, Isohexyl-, 2-Ethylbutyl-, 3,3-Dimethyl-butyl-, 1-Methylhexyl-, 4-Methyl-hexyl-, 5-Methyl-hexyl-, 2-Ethyl-hexyl-, 3,5-Dimethyl-hexyl- und 3-Methyl-octyl-Rest.

Geradkettige oder verzweigte, ungesättigte Alkylreste sind bevorzugt der Allyl-, 3-Butenyl-, 3-Pentenyl-, 2-, 3-, 4- oder 5-Hexenyl-, 9-Decenyl-, 2-Methylallyl-, 3,7-Dimethyl-6-octenyl-, 2-Propinyl-, 2-Butinyl- und 2-Hexinyl-Rest.

Gesättigte und ungesättigte cyclische Alkylreste des Substituenten R sind Carbocyclen mit 3—8 Kohlenstoffatomen. Als gesättigte Carbocyclen kommen vorzugsweise der Cyclopentyl-, Cyclohexyl- und Cycloheptyl-Rest in Frage, als ungesättigte Carbocyclen der 1-, 2- oder 3-Cyclohexen-1-yl-Rest.

Ferner sind Gegenstand der Erfindung sämtliche stereoisomeren Formen einer Verbindung der allgemeinen Formel I, die aufgrund der bei manchen Verbindungen vorhandenen asymmetrischen Kohlenstoffatome oder Doppelbindungen (C=C; C=N) auftreten können.

Die Verbindungen der allgemeinen Formel I können nach an sich bekannten Verfahren hergestellt werden, vorzugsweise indem man ein Hydrazin der allgemeinen Formel II

$$R—X—NH—NH_2 \quad (II),$$

in der R und X die obige Bedeutung haben,

mit einem Propionsäure-Derivat der Formel III

$$CH_3—C(Y,Y')—COR' \quad (III),$$

in der Y und Y' Halogen- oder Alkoxy-Gruppen oder zusammen ein Sauerstoffatom darstellen und R' Hydroxy, eine niedere Alkoxy- oder eine gegebenenfalls substituierte Amingruppe bedeutet, umsetzt und anschließend gegebenenfalls die Säure in ihr Salz, einen Ester oder ein Amid überführt bzw. die Säure aus ihren Derivaten freisetzt.

Halogen der Reste Y und Y' der Formel III bedeutet Fluor, Chlor, Brom und Jod, vorzugsweise jedoch Chlor und Brom. Alkoxygruppen der Reste Y, Y' und R' sind Gruppen mit 1—4 Kohlenstoffatomen, bevorzugt ist die Methoxy- und Ethoxygruppe.

Bei diesem Verfahren wird das substituierte Hydrazin II oder ein entsprechendes Salz in einem geeigneten polaren Lösungsmittel (z.B. Wasser, einem niederen Alkohol oder Essigsäure) mit einem Propionsäure-Derivat III oder vorzugsweise mit dessen Salzen versetzt und gegebenenfalls mit Hilfe eines Puffers, z.B. Natriumacetat, auf ein schwach saures pH gebracht. Die Reaktion läuft bei Raumtemperatur ab, kann jedoch unter Erwärmen durchgeführt werden. Die Hydrazone I können als schwerlösliche Verbindungen aus dem Reaktionsmedium abfiltriert oder mit geeigneten Lösungsmitteln extrahiert werden, z.B. unpolaren Lösungsmitteln.

Gegebenenfalls kann auch in einem Eintopfverfahren das substituierte Hydrazin II z.B. aus einem Amin mit Hydroxylamino-O-sulfonsäure hergestellt und nach Zugabe des Propionsäure-Derivates III das gewünschte Hydrazon ausgefällt werden.

Die substituierten Hydrazine bzw. ihre Salze sind zum Teil neue Verbindungen. Eine Reindarstellung ist im allgemeinen nicht erforderlich, so daß die anfallenden Rohprodukte eingesetzt werden können. Die Hydrazine können nach an sich bekannten Verfahren hergestellt werden, beispielsweise durch Umsetzung von Hydrazin mit entsprechenden Alkylhalogeniden.

Als physiologisch unbedenkliche Salze kommen insbesondere Alkali-, Erdalkali- oder Ammoniumsalze (sowie gegebenenfalls Salze mit ebenfalls blutzuckersenkenden Biguaniden) in Frage. Die Herstellung dieser Salze erfolgt in an sich bekannter Weise, beispielsweise durch Umsetzung mit den entsprechenden freien Basen, Carbonaten oder Alkoholaten.

Die bei dem oben aufgeführten Verfahren als Zwischenprodukte auftretenden Ester können isoliert oder gegebenenfalls direkt zu den entsprechenden Carbonsäuren verseift werden. Umgekehrt können die erhaltenen Carbonsäuren wieder nach an sich bekannten Methoden zu den gewünschten Estern umgesetzt werden. Die Verseifungen der Ester werden vorzugsweise in alkalischem Medium durchgeführt.

Als Ester der Carbonsäuren der allgemeinen Formel I sind im Sinne der Erfindung prinzipiell die Reaktionsprodukte der Carbonsäure mit Alkoholen zu verstehen. Bevorzugt sind jedoch die niederen einwertigen Alkohole wie Methanol, Ethanol, Propanol oder Isopropanol.

Die erfindungsgemäßen Amide der allgemeinen Formel I können nach an sich bekannten Methoden aus den Carbonsäuren oder ihren reaktiven Derivaten durch Umsetzung mit Aminen hergestellt werden. Als Aminkomponenten kommen z.B. Ammoniak, Mono- und Dialkylamine sowie Aminosäuren in Frage, wobei p-Aminobenzoesäure, Anthranilsäure, Phenylalanin, $\alpha$- und $\beta$-Alanin, Serin, Valin, Glycin und Arginin zu nennen sind.

Als erfindungsgemäße blutzuckersenkende Zubereitungen kommen alle üblichen oralen und parenteralen Applikationsformen in Frage, beispielsweise Tabletten, Kapseln, Dragées, Sirupe, Lösungen, Suspensionen, Tropfen, Suppositorien etc. Zu diesem Zweck vermischt man den Wirkstoff mit festen oder flüssigen Trägerstoffen und bringt sie anschließend in die gewünschte Form. Feste Trägerstoffe sind z.B. Stärke, Lactose, Methylcellulose, Talkum, hochdisperse Kieselsäure, höher-molekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole). Für die orale Applikation geeignete Zubereitungen können gewünschtenfalls Geschmacks- und Süßstoffe enthalten.

Als Injektionsmedium kommt vorzugsweise Wasser zur Anwendung, welches die bei Injektionslösungen üblichen Zusätze wie Stabilisierungsmittel, Lösungsvermittler und/oder Puffer enthält. Derartige Zusätze sind z.B. Acetat- oder Tartrat-Puffer, Ethanol, Komplexbildner (wie Ethylendiamin-tetraessigsäure und deren nicht-toxische Salze) oder hochmolekulare Polymere (wie flüssiges Polyethylenoxid) zur Viskositätsregulierung.

Bevorzugt im Sinne der vorliegenden Anmeldung sind außer den in den Beispielen genannten Verbindungen noch die folgenden Säuren bzw. ihre Salze.

2-(2-Butinylhydrazono)-propionsäure
2-(3,5-Dimethylhexylhydrazono)-propionsäure
2-(3,7-Dimethyl-6-octenylhydrazono)-propionsäure
2-[2-(1-Cyclohexen-1-yl)-ethylhydrazono]-propionsäure
2-(3-Cyclohexyl-2-methyl-2-propenylhydrazono)-propionsäure
2-[2-(Cyclohexen-1-yl)-ethylhydrazono]-propionsäure
2-(2-Propinylhydrazono)-propionsäure

2-(2-Hexinylhydrazono)-propionsäure
2-(3-Pentenylhydrazono)-propionsäure
2-[3-(3-Cyclohexen-1-yl)-2-methyl-2-propenylhydrazono]propionsäure

In den nachfolgenden Beispielen wird die Erfindung näher erläutert:

Beispiel 1

*2-(2-Cyclohexyl-ethylhydrazono)-propionsäure*

2.7 g (2-Cyclohexylethyl)-hydrazin-sulfat (Fp. 185—187°C, Zers.) werden in 50 ml Wasser gelöst und unter Rühren bei Raumtemperatur mit der Lösung von 1,1 g Brenztraubensäure und 3,0 g Natrimacetat (Trihydrat) in 10 ml Wasser versetzt. Dabei scheidet sich zunächst ein Öl ab, das bald kristallisiert. Die Substanz wird abgesaugt, unter Zugabe von 6 ml 2 N Natronlauge in Wasser gelöst und langsam mit 2 N Salzsäure angesäuert. Die zuerst ausfallende Teilmenge enthält Verunreinigungen, wird abgesaugt und verworfen. Die durch weiteres Ansäuern ausgefällte Hauptmenge ist rein und wird im Exsiccator über Calciumchlorid getrocknet.

Ausbeute 1,6 g (67% d. Th.); Fp. 47°C.

In analoger Weise erhält man durch Umsetzung von Brenztraubensäure

a) mit Isobutylhydrazin-sulfat (Fp. 127°C, Zers.)

*2-(Isobutylhydrazono)-propionsäure*
Fp. 88°C (aus Isooctan)

b) mit Methylallylhydrazin-oxalat (Fp. 160—161°C, Zers.)

*2-(Methylallylhydrazono)-propionsäure*
Fp. 68—69°C (aus Isooctan + Toluol)

c) mit Propylhydrazin-oxalat

*2-(Propylhydrazono)-propionsäure*
Fp. 56—58°C (aus Ligroin + Toluol)

d) mit Isopentylhydrazin-hydrochlorid (Fp. 128—130°C, Zers.)

*2-(Isopentylhydrazono)-propionsäure*
Fp. 48—50°C (aus Isooctan)

e) mit Octylhydrazin-hydrochlorid

*2-(Octylhydrazono)-propionsäure*
Fp. 37—38°C

f) mit (Cyclohexylmethyl)-hydrazin-hydrochlorid

*2-(Cyclohexylmethyl-hydrazono)-propionsäure*
Fp. 81—82°C (aus Isooctan + Toluol)

g) mit Isopropylhydrazin-hydrochlorid

*2-(Isopropylhydrazono)-propionsäure*
Fp. 83—84°C (aus Ligroin)

h) mit Allylhydrazin-hydrochlorid

*2-(Allylhydrazono)-propionsäure*
Fp. 47—48°C (aus Methylenchlorid)

i) mit Butylhydrazin-sulfat

*2-(Butylhydrazono)-propionsäure*
Fp. 56—57°C (aus Cyclohexan)

j) mit sec-Butylhydrazin-sulfat

*2-(sec-Butylhydrazono)-propionsäure*
Fp. 67—68°C (mit Soda/HCl umgefällt)

k) mit tert-Butylhydrazin-hydrochlorid

*2-(tert-Butylhydrazono)-propionsäure*
Fp. 99—100°C (Isopropanol + Wasser)

l) mit Hexylhydrazin-hydrochlorid

*2-(Hexylhydrazono)-propionsäure*
Fp. 44°C (mit Soda/HCl umgefällt)

m) mit 4-Methylpentylhydrazin-hydrochlorid (Rohprodukt)

*2-(4-Methylpentylhydrazono)-propionsäure*
ölig

n) mit 2-Ethyl-butylhydrazin-hydrochlorid (Fp. 118—125°C, Rohprodukt)

*2-(2-Ethylbutylhydrazono)-propionsäure*
Fp. 83—86°C (aus Isopropanol + Wasser)

o) mit 5-Hexenyl-hydrazin-hydrochlorid (Fp. 130—135°C, Rohprodukt)

*2-(5-Hexenyl-hydrazono)-propionsäure*
ölig

p) mit Heptylhydrazin-hydrochlorid

*2-Heptylhydrazono)-propionsäure*
Fp. 48—49°C (aus Ligroin)

q) mit (2-Cyclopentylethyl)-hydrazin-hydrochlorid (Fp. 160°C, Rohprodukt)

*2-(2-Cyclopentylethylhydrazono)-propionsäure*
Fp. 64—65°C (aus Ether + Ligroin)

r) mit 2-(3-Cyclohexen-1-yl)-ethylhydrazin-hydrochlorid (Fp. 98—138°C, Rohprodukt)

*2-[2-(3-Cyclohexen-1-yl)-ethylhydrazono]-propionsäure*
Fp. 67—68°C (aus Isopropanol + Wasser)

s) mit 2-Cycloheptylethylhydrazin-hydrochlorid (Fp. 132—145°C, Rohprodukt)

*2-(2-Cycloheptylethylhydrazono)-propionsäure*
(Fp. 59—61°C (aus Cyclohexan)

t) mit Decylhydrazin-hydrochlorid (Fp. 94°C, Zers.)

*2-(Decylhydrazono)-propionsäure*
Fp. 48—49°C (aus Isooctan)

u) mit Nonylhydrazin-hydrochlorid (Fp. 115°C, Zers.)

*2-(Nonylhydrazono)-propionsäure*
Fp. 46—47°C (aus Isooctan)

v) mit (3-Cyclohexyl-propyl)-hydrazin-hydrochlorid (Fp. 220—225°C)

*2-(3-Cyclohexylpropylhydrazono)-propionsäure*
ölig

Beispiel 2

*Natrium-2-(pentylhydrazono)-propionat*

2.3 g Pentylhydrazin-hydrochlorid werden in 10 ml Wasser gelöst und mit der Lösung von 1.5 g Brentztraubensäure und 2.2 g Natriumacetat in 5 ml Wasser gemischt. Es bildet sich ein Öl. Man rührt etwa eine Stunde, extrahiert das Öl mit Methylenchlorid, wäscht diese Lösung mit Wasser, trocknet sie mit Natriumsulfat und verdampft das Methylenchlorid. Den öligen Rückstand löst man in 8 ml Ethanol und gibt unter Rühren 3,0 ml einer 30-proz. Natriummethylat-Lösung zu. Das natrium-2-(pentylhydrazono)-propionat scheidet sich ab, wird abgesaugt und zuerst mit wenig Ethanol, dann mit Ether gewaschen.

Ausbeute 1,9 g (59% d. Th.); Fp. 225—228°C, Zers.

In analoger Weise erhält man durch Umsetzung von Brenztraubensäure

a) mit (2-Cyclohexylethyl)-hydrazin-sulfat und anschließender Darstellung des Natriumsalzes

*Natrium-2-(2-cyclohexyl-ethylhydrazono)-propionat*
Fp. 230—233°C, Zers.

b) mit (3-Cyclohexyl-propyl)-hydrazin-hydrochlorid (Fp. 220—225°C) und anschließender Darstellung des Natriumsalzes

*Natrium-2-(3-cyclohexyl-propyl-hydrazono)-propionat*
Fp. 224—226°C, Zers.

Beispiel 3

In analoger Weise wie in Beispiel 1 beschrieben erhält man durch Umsetzung von Brenztraubensäure

a) mit 9-Decenylhydrazin-hydrochlorid (Rohprodukt)

*2-(9-Decenylhydrazono)-propionsäure*
Fp. 39—41°C (Hexan)

b) mit 2-Hexenylhydrazin-oxalat (Fp. 166—167°C)

*2-(2-Hexenylhydrazono)-propionsäure*
ölig

c) mit Methylhydrazin-sulfat

*2-(Methylhydrazono)-propionsäure*
Fp. 89—91°C (Isopropanol + Isooctan)

d) mit 5-Methylhexylhydrazin-hydrochlorid (Fp. 184—188°C, Rohprodukt)

*2-(5-Methylhexylhydrazono)-propionsäure*
ölig

Beispiel 4

*2-(Decylhydrazono)-propionsäureamid*

6,5 g Decylhydrazindihydrochlorid werden in 30 ml Wasser gelöst und mit der Lösung von 2,3 g Brenztraubensäureamid in 40 ml Wasser versetzt. Durch Zugabe von verdünnter Natronlauge wird der pH auf ca. 3 eingestellt und die Lösung für 16 Stunden in den Kühlschrank gestellt; dabei kristallisiert die gewünschte Substanz aus, wird abgesaugt und aus Isooctan umkristallisiert.

Ausbeute: 3,3 g (51,6% d. Th.); Fp. 64°C (Zers.).

Beispiel 5

*2-(Heptylhydrazono)-propionsäureethylester*

4,0 g Heptylhydrazindihydrochlorid werden in 40 ml Ethanol gelöst und 2,3 g Brenztraubensäureethylester zugegeben. Die Lösung wird 20 Minuten bei Zimmertemperatur aufbewahrt, dann mit 160 ml Wasser versetzt und das sich abscheidende Öl mit Ether extrahiert. Die Etherlösung wird mehrmals mit 2 N Natriumcarbonatlösung und danach mit Wasser gewaschen, mit Natriumsulfat getrocknet und eingeengt. Der als Rückstand erhaltene Ester kristallisiert nicht.

Ausbeute: 3,7 g (82,4% d. Th.).

In analoger Weise erhält man

a) unter Verwendung von Nonylhydrazindihydrochlorid und Brenztraubensäuremethylester

*2-(Nonylhydrazono)-propionsäuremethylester*
ölig

Beispiel 6

In analoger Weise wie in Beispiel 1 beschrieben erhält man durch Umsetzung von Brenztraubensäure

a) mit 4-Hexenylhydrazinhydrochlorid, freie Verbindung $Kp_{14}$ 82—89°C, HCl-Salz hygroskopisch

*2-(4-Hexenylhydrazono)-propionsäure*
ölig
die Verbindung enthält 0,6 mol Wasser

b) mit Octadecylhydrazin-dihydrochlorid Fp. 264°C (Zers.) nach Sintern ab 88°C

*2-(Octadecylhydrazono)-propionsäure*
Fp. 80°C (aus Isooctan)

c) mit 3-Butenylhydrazin-hydrochlorid (Rohprodukt)

*2-(3-Butenylhydrazono)-propionsäure*
ölig

d) mit 3,3-Dimethylbutylhydrazin-hydrochlorid Fp. 213—214°C

*2-(3,3-Dimethylbutylhydrazono)-propionsäure*
Fp. 95°C (aus Isooctan)

e) mit 3-Methyloctylhydrazin-oxalat Fp. 190°C (Zers.)

*2-(3-Methyl-octylhydrazono)-propionsäure*
ölig

f) mit 3-Cyclohexyl-2-propenylhydrazin-hydrochlorid Fp. 165°C (Zers.)

*2-(3-Cyclohexyl-2-propenylhydrazono)-propionsäure*
ölig

g) mit Dodecylhydrazin-dihydrochlorid Fp. 210°C (Zers.) nach Sintern ab 80°C

*2-(Dodecylhydrazono)-propionsäure*
Fp. 60—61°C (mit Isooctan)

h) mit 4-Methylhexylhydrazin-dihydrochlorid (hygroskopisch; freie Base; $Kp_{13}$: 86—88°C)

*2-(4-Methylhexylhydrazono)-propionsäure*
ölig

i) mit 1-Methylhexylhydrazin-oxalat Fp. 95°C

*2-(1-Methylhexylhydrazono)-propionsäure*
ölig

j) mit (2-Ethylhexyl)-hydrazin (freie Base; $Kp_{0,1}$: 56—60°C)

*2-(2-Ethylhexylhydrazono)-propionsäure*
ölig

k) mit (3-Hexenyl-hydrazin-hydrochlorid Fp. 166—167°C

*2-(3-Hexenylhydrazono)-propionsäure*
ölig

l) mit (3-Cyclopentyl-propyl)-hydrazin-hydrochlorid Fp. 197—202°C

*2-(3-Cyclopentyl-propylhydrazono)-propionsäure*
ölig

Beispiel 7

In analoger Weise wie im Beispiel 2 beschreiben erhält man durch Umsetzung von Brenztraubensäure

a) mit 3-Cyclohexyl-2-methyl-2-propenylhydrazin-hydrochlorid (ölig)

und anschließender Darstellung des Natriumsalzes

*Natrium-2-(3-cyclohexyl-2-methyl-2-propenylhydrazono)-propionat*
Fp. 266—268°C (Zers.) (aus Ethanol)

**Patentansprüche**

1. 2-Hydrazono-propionsäure-Derivate der allgemeinen Formel I

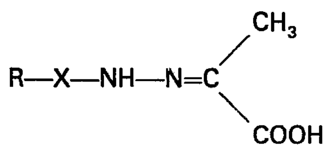

(I),

in der

*R* ein geradkettiger, verzweigter oder cyclischer, gesättigter oder ungesättigter Alkyl-Rest und

*X* ein Valenzstrich oder eine geradkettige oder verzweigte, gesättigte oder ungesättigte Alkylen-Gruppe mit 1—4 Kohlenstoffatomen ist,

wobei für den Fall, daß X einen Valenzstrich darstellt, R kein gesättigter Cycloalkyl-Rest sein darf, deren physiologisch unbedenkliche Salze, Ester und Amide.

2. Verfahren zur Herstellung von 2-Hydrazono-propionsäure-Derivaten gemäß Anspruch 1, dadurch gekennzeichnet, daß man ein Hydrazin der allgemeinen Formel II

$$R—X—NH—NH_2 \quad (II),$$

in der

R und X die im Anspruch 1 angegebene Bedeutung haben, mit einem Propionsaure-Derivat der Formel III

$$CH_3—C(Y, Y')—COR' \quad (III),$$

in der

Y und Y' Halogen oder Alkoxy bedeuten oder zusammen ein Sauerstoffatom darstellen und

R' Hydroxy, eine niedere Alkoxy- oder eine gegebenenfalls substituierte Aminogruppe bedeutet, umsetzt,

und anschließend gegebenenfalls die erhaltene Säure in ihr Salz, einen Ester oder ein Amid überführt bzw. die Säure aus ihren Derivaten freisetzt.

3. Arzneimittel enthaltend einen Wirkstoff der Formel I sowie an sich bekannte pharmakologisch verträgliche Trägerstoffe.

4. Verbindungen gemäß Anspruch 1 oder deren Salze zur Verwendung in der Behandlung von Diabetes.

**Claims**

1. 2-Hydrazonopropionic acid derivatives of the general formula I

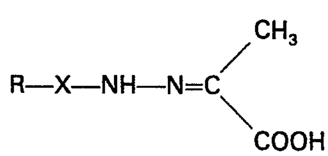

(I)

in which R is a straight-chained, branched or cyclic, saturated or unsaturated alkyl radical and X is a

valency bond or a straight-chained or branched, saturated or unsaturated alkylene group with 1—4 carbon atoms, whereby, when X represents a valency bond, R cannot be a saturated cycloalkyl radical; their physiologically acceptable salts, esters and amides.

2. A process for the preparation of 2-hydrazono-propionic acid derivatives according to claim 1, characterised in that one reacts a hydrazine of the general formula (II)

$$R—X—NH—NH_2 \quad \text{(II)},$$

in which R and X have the meaning given in claim 1, with a propionic acid derivative of the formula (III)

$$CH_3—C(Y,Y')—COR' \quad \text{(III)}$$

in which Y and Y' signify halogen or alkoxy or together represent an oxygen atom and R' signifies hydroxyl, a lower alkoxy or a possibly substituted amino group, and subsequently one possibly converts the acid obtained into its salt, an ester or an amide or liberates the acid from its derivatives.

3. Medicaments containing an active material of formula I, as well as per se known pharmacologically acceptable carrier materials.

4. Compounds according to claim 1 or their salts for use in the treatment of diabetes.

**Revendications**

1. Dérivés de l'acide 2-hydrazonopropionique de formule générale I

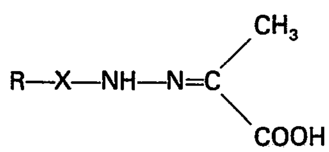

(I)

dans laquelle:

R est un reste alkyle à chaîne droite, ramifiée ou cyclique, saturé ou insaturé et,

X est un trait de valence ou un groupe alkylène à chaîne droite ou ramifiée, saturé ou insaturé ayant de 1 à 4 atomes de carbone, et dans le cas où X est un trait de valence, R ne doit pas être un reste cyclo-alkyle saturé,

ainsi que leurs sels, amides et esters physiologiquement irréprochables.

2. Procédé pour la préparation de dérivés de l'acide 2-hydrazonopropionique selon la revendication 1 caractérisé en ce que l'on fait réagir une hydrazine de formule générale II.

$$R—X—NH—NH_2 \quad \text{(II)}$$

dans laquelle:

R et X ont la signification indiquée dans la revendication 1, avec un dérivé de l'acide propionique de formule III.

$$CH_3—C(Y,Y')—COR' \quad \text{(III)},$$

dans laquelle:

Y et Y' sont un atome d'halogène ou un groupe alcoxyle ou représentent ensemble un atome d'oxygène, et

R' est une fonction hydroxyle, un groupe alcoxyle inférieur ou un groupe aminogène éventuellement substitué, et en ce qu'en-suite, éventuellement, on transforme l'acide obtenu en un sel, un ester ou un amide, ou bien on libère l'acide de ses dérivés.

3. Médicament ayant une teneur en substance active de la formule I et en substances de support pharmacologiquement tolérables connues.

4. Dérivés selon la revendication 1 ou leurs sels pour l'utilisation dans la traitement du diabète.